# EUROPÄISCHE PATENTSCHRIFT

(11) **EP 3 292 855 B1**
(45) Veröffentlichungstag und Bekanntmachung des Hinweises auf die Patenterteilung: **03.06.2026**
(21) Anmeldenummer: 17187367.2
(22) Anmeldetag: 22.08.2017
(51) Int. Cl.: A61J 1/14

(54) **VERSCHLUSSKAPPE FÜR EINEN KARTUSCHENFÖRMIGEN BEHÄLTER**
CLOSURE CAP FOR A CARTRIDGE SHAPED CONTAINER.
CAPUCHON DE FERMETURE POUR UN CONTENANT EN FORME DE CARTOUCHE

(30) Priorität: 30.08.2016 DE 102016116098
(43) Veröffentlichungstag der Anmeldung: 14.03.2018
(73) Patentinhaber: B. Braun Avitum AG, 34212 Melsungen (DE)
(72) Erfinder: KLAUS, Dinah, 48147 Münster (DE); WESSELER, Matthias, 49326 Melle (DE); KATHOL, Johannes, 6130 Willisau (CH)
(74) Vertreter: Winter, Brandl - Partnerschaft mbB

(56) Entgegenhaltungen:
- EP-A1- 2 712 603
- EP-B1- 0 092 884
- EP-B1- 1 114 781
- EP-B1- 2 712 603
- WO-A1-2015/145902
- DE-A1- 4 029 832
- FR-A1- 2 564 071
- JP-A- 2010 143 081
- US-A- 4 015 400
- US-A- 4 448 318
- US-A1- 2005 051 572

## Beschreibung

Die Erfindung betrifft eine Verschlusskappe, welche zum Aufstecken an einem, insbesondere rohrstutzenförmigen, Anschlussport/ Konnektor eines kartuschenförmigen Behälters/ einer Kartusche vorbereitet ist und in einem montierten Zustand einem Verschließen des Anschlussports dient, mit einem, insbesondere rund/ kreisförmig ausgebildeten, Deckflächenabschnitt, einem sich, insbesondere senkrecht und zylindrisch, an den Deckflächenabschnitt anschließenden Mantelflächenabschnitt, und einem sich an den Mantelflächenabschnitt anschließenden, insbesondere ringförmigen, Flanschabschnitt. Außerdem betrifft die Erfindung einen kartuschenförmigen Behälter/ eine Kartusche für eine extrakorporale Blutbehandlungsmaschine, welcher/ welche zumindest einen einen Fluideinlass und/oder einen Fluidauslass bildenden, insbesondere rohrstutzenförmigen, Anschlussport und zumindest eine Verschlusskappe zum Verschließen des zumindest einen Anschlussports aufweist.

Der Therapieerfolg der verschiedensten Dialyseverfahren beruht unter anderem auf dem Einsatz verschiedener Puffersubstanzen, mit Hilfe derer ein veränderter Säure-Basen-Haushalt von niereninsuffizienten Patienten korrigiert werden kann. Vor dem Hintergrund, dass sich der Säure-Basen-Haushalt nicht durch Diffusion während der Dialyse korrigieren lässt, ist häufig eine Zufuhr von Puffersubstanzen erforderlich. Zum Ausgleichen des Gefälles zwischen Säuren und Basen eignet sich insbesondere Bikarbonat bzw. eine Bikarbonat-Pufferlösung. Die Bikarbonat-Pufferlösung wird für die Dialyse bevorzugt erst unmittelbar vor bzw. während der Behandlung hergestellt. Dabei wird ein Behälter, insbesondere ein kartuschenförmiger Behälter/ eine Kartusche, welcher/ welche ein Bikarbonatkonzentratpulver enthält, an eine Fluidquelle, beispielsweise eine Wasserquelle angeschlossen, wobei das den Behälter durchströmende Wasser das in dem Behälter befindliche Bikarbonatkonzentratpulver auflöst und dieses in die Dialysierflüssigkeit ausschwemmt.

Die bekannten kartuschenförmigen Behälter/ Kartuschen weisen dabei grundsätzlich einen Fluideinlass-Anschlussport/ Konnektor und einen Fluidauslass-Anschlussport/ Konnektor auf, an welche jeweils eine Fluideinlassleitung und eine Fluidauslassleitung anbringbar sind. Die Anschlussports/ Konnektoren bestehen dabei grundsätzlich aus kurzen, von dem Behälter axial hervorragenden Rohrabschnitten oder Rohrstutzen. Um eine weltweit einheitliche, das heißt temperatur-, druck- und luftfeuchtigkeitsunabhängige Haltbarkeit bei Bikarbonat-Kartuschen sicherstellen bzw. garantieren zu können, ist es aus dem Stand der Technik bekannt, Verschlusskappen mit Dichtfunktion auf die rohrstutzenförmigen Anschlussports/ Konnektoren aufzustecken bzw. aufzuschrauben.

Beispielsweise ist aus der EP 0 532 835 B1 ein kartuschenförmiger Behälter/ eine Kartusche mit einem Anschlussport, auf welchem eine Verschlusskappe mit Dichtfunktion aufgesetzt bzw. aufgesteckt ist, bekannt. Ferner offenbart die DE 42 17 352 C2 eine Verschlusskappe, welche auf einem Anschlussport eines kartuschenförmigen Behälters/ einer Kartusche aufgeschraubt ist und eine Silikondichtung beinhaltet. Die Verschlusskappen der EP 0 532 835 B1 und DE 42 17 352 C2 weisen jedoch den Nachteil auf, dass sie nicht an dem Behälter/ der Kartusche/ dem Anschlussport versiegelt sind. Ein Anwender kann somit nicht feststellen, ob der Behälter/ die Kartusche noch original verschlossen ist, insbesondere einen Originalitätsverschluss aufweist, oder bereits geöffnet wurde. Die Kappe der DE 42 17 352 C2 weist zudem Handhabungsnachteile auf, da sie auf den Anschlussport aufgeschraubt werden muss und nicht aufgesteckt werden kann.

Um sicherstellen zu können, dass der kartuschenförmige Behälter/ die Kartusche noch original verschlossen ist bzw. einen Originalitätsverschluss aufweist, ist es aus dem Stand der Technik grundsätzlich bekannt, den rohrstutzenförmigen Anschlussport des Behälters/ der Kartusche mit einer Folie/ einem Foliensiegel abzudichten. Wird jedoch lediglich eine Folie zum Versiegeln an dem Anschlussport angebracht, hat dies den Nachteil, dass diese bei einer Lagerung und/ oder einem Transport beschädigt und durchstoßen werden kann. Daher wird beispielsweise in der WO 97/26941 A1 vorgeschlagen, auf einem folienversiegelten Anschlussport zusätzlich eine Verschlusskappe zum Schutz des Foliensiegels aufzuschrauben. Dies verursacht beim Öffnen des Behälters/ der Kartusche jedoch einen zusätzlichen Handhabungsschritt und erfordert ein zusätzliches Entsorgen der Folie.

In der US 5,807,345 A ist eine Kappe zum Verschließen und Versiegeln einer Spritze offenbart, welche einen formschlüssig in einem Hülsenabschnitt der Spritze aufgenommenen Flanschabschnitt aufweist. Wird die Kappe der US 5,807,345 A jedoch von der Spritze entfernt und anschließend wieder an dieser befestigt bzw. angebracht, kann ein Anwender von außen und auch beim erneuten Entfernen der Kappe nicht erkennen, dass diese bereits zuvor entfernt worden war und somit kein Originalitätsverschluss mehr vorliegt.

In der DE 26 45 563 A1 ist eine Verschlusskappe mit einer gesicherten Aufreißöffnung offenbart, welche einen Sollbruch-/ -rissabschnitt, an welchem die Verschlusskappe beim Entfernen derselben von einem Behälter definiert reißen/ brechen soll, an einem Mantelflächenabschnitt der Verschlusskappe aufweist. Das Vorsehen eines perforierten/ eine Perforation aufweisenden Sollbruch-/ rissabschnitts an einem Mantelflächenabschnitt der Verschlusskappe hat jedoch den Nachteil, dass ein Anwender nicht auf den ersten Blick feststellen kann, ob das Siegel noch ungebrochen ist oder die Kartusche bereits geöffnet war. Außerdem besteht bei der Verschlusskappe der DE 26 45 563 A1 die Gefahr, wenn die Verschlusskappe zur Entsorgung, insbesondere zum Verhindern eines Abtropfens der in dem Konzentratbehälter befindlichen Restlösung, erneut auf den Anschlussport gesetzt wird, dass diese nicht mehr in ausreichender Weise an dem Anschlussport hält und beim Transport und/oder bei der Lagerung herunterfällt.

Die US 2005/051572 A1 und die EP 2 712 603 A1 offenbaren jeweils eine Verschlusskappe, welche zum Befestigen an einem Anschlussport eines kartuschenförmigen Behälters vorbereitet ist und in einem montierten Zustand einem Verschließen des Anschlussports dient, mit einem runden Deckflächenabschnitt, einem sich senkrecht an den Deckflächenabschnitt anschließenden, im Wesentlichen zylindrisch verlaufenden Mantelflächenabschnitt, und einem sich an den Mantelflächenabschnitt anschließenden, sich endseitig des Mantelflächenabschnitts von dem Mantelflächenabschnitt senkrecht und radial nach außen weg erstreckenden Flanschabschnitt, wobei ein Sollbruch-/ Rissabschnitt an dem Mantelflächenabschnitt vorgesehen ist.

Aus der US 4 225 050 A, der EP 0 941 938 A2, der JP 2003 112 752 A, der JP 2010 143 081 A, der US 4 448 318 A und der EP 1 114 781 A2 sind Schraubkappen bekannt, an welchen an einem Flanschabschnitt ein Sollbruch-/ Rissabschnitt vorgesehen ist. Eine weitere Verschlusskappe ist aus der FR 2 564 071 A1 bekannt. Aus der EP 0 092 884 A1 ist ein Dichtring mit einem Sollbruch-/ Rissabschnitt bekannt.

Vor diesem Hintergrund ist es Aufgabe der vorliegenden Erfindung, die Nachteile aus dem Stand der Technik zu vermeiden oder wenigstens zu mildern. Insbesondere soll eine Verschlusskappe für einen, insbesondere kartuschenförmigen, Behälter/ eine Kartusche bereitgestellt werden, welche in einfacher Weise handhabbar ist und einen für einen Anwender schnell und einfach zu überprüfenden, versiegelten Originalitätsverschluss bereitstellt. Insbesondere ist es Aufgabe der Erfindung, eine sofortige Sichtprüfung des Originalitätsverschlusses zu ermöglichen. Zudem soll ein erneutes Anbringen der Verschlusskappe zu Entsorgungszwecken verbessert werden.

Diese Aufgabe wird durch eine Verschlussklappe nach Anspruch 1 und einen kartuschenförmigen Behälter/ eine Kartusche nach Anspruch 9 gelöst. Vorteilhafte Ausführungsformen und Weiterbildungen sind in den Unteransprüchen beansprucht und/ oder nachfolgend erläutert.

Die Erfindung betrifft zunächst eine, insbesondere eine stoßdämpfende Wirkung aufweisende, Verschlusskappe, welche zum Befestigen, insbesondere zum Aufstecken, an einem, insbesondere rohrstutzenförmigen, Anschlussport/ Konnektor eines kartuschenförmigen Behälters/ einer Kartusche vorbereitet ist und in einem montierten Zustand einem Verschließen des Anschlussports dient, mit einem, insbesondere runden/ rund/kreisförmig ausgebildeten, Deckflächenabschnitt, einem sich, insbesondere senkrecht, an den Deckflächenabschnitt anschließenden, bevorzugt im Wesentlichen zylindrisch verlaufenden, Mantelflächenabschnitt, und einem sich an den Mantelflächenabschnitt anschließenden, insbesondere ringförmigen, bevorzugt sich endseitig des Mantelflächenabschnitts von dem Mantelflächenabschnitt senkrecht und radial nach außen weg erstreckenden, Flanschabschnitt, wobei der Flanschabschnitt einen Versiegelungsabschnitt, über welchen die Verschlusskappe an dem kartuschenförmigen Behälter/ der Kartusche fixierbar und versiegelbar ist, und einen Sollbruch-/ -rissabschnitt, an welchem die Verschlussklappe beim Entfernen der Verschlusskappe von dem kartuschenförmigen Behälter/ der Kartusche vordefiniert reißt und/oder bricht, aufweist und/oder ausbildet.

Es wird somit in anderen Worten erfindungsgemäß eine Verschlusskappe in der Form eines sacklochartig ausgenommenen Zylinders bereitgestellt, welche an ihrem offenen Ende einen, sich insbesondere von dem Mantelflächenabschnitt radial, vorzugsweise senkrecht, nach außen erstreckenden, Flanschabschnitt aufweist. Die Verschlusskappe kann in einfacher Weise durch Aufstecken auf einen Anschlussport eines kartuschenförmigen Behälters/ einer Kartusche, welcher/ welche Pulverpartikel, etwa Bikarbonat, Natriumchlorid oder Ähnliches, beinhaltet, angebracht werden, so dass insbesondere ein geschlossenes, das heißt versiegeltes und/oder abgedichtetes, Kartuschensystem mit Hilfe bzw. durch die erfindungsgemäße Verschlusskappe/ Kappe/ Abdeckkappe entstehen kann. Die Verschlusskappe hat zudem eine Schutzfunktion für den Anschlussport und verhindert, dass dieser in irgendeiner Weise beschädigt werden kann.

Der Flanschabschnitt weist dabei einen Versiegelungsabschnitt, insbesondere Siegelring, auf, über welchen die Verschlusskappe an dem kartuschenförmigen Behälter/ der Kartusche bzw. dem Anschlussport versiegelt und befestigt/ fixiert werden kann. Der Versiegelungsabschnitt dient in anderen Worten einem Befestigen und einem Versiegeln der Verschlusskappe an dem kartuschenförmigen Behälter/ der Kartusche/ dem Anschlussport. Im an dem kartuschenförmigen Behälter/ der Kartusche fixierten/ befestigten Zustand der Verschlusskappe wird dadurch ein Originalitätsverschluss bereitgestellt. Der Versiegelungsabschnitt ist vorzugsweise an einer radialen Außenseite des Flanschabschnitts vorgesehen. Der Versiegelungsabschnitt/ der Siegelring kann erfindungsgemäß zinnenförmig ausgebildet sein.

Wenn nun der Flanschabschnitt neben dem/ zusätzlich zu dem Versiegelungsabschnitt einen Sollbruch-/ -rissabschnitt, an welchem die Verschlusskappe beim Entfernen der Verschlusskappe von dem kartuschenförmigen Behälter/ der Kartusche vordefiniert reißt und/oder bricht, aufweist und/oder ausbildet, dann kann ein Anwender sofort bei Betrachtung des sich radial nach außen erstreckenden Flanschabschnitts, das heißt auf den ersten Blick, erkennen, ob der kartuschenförmige Behälter/ die Kartusche noch original verschlossen ist oder bereits geöffnet wurde. In anderen Worten ist der Kerngedanke der Erfindung der, dass ein Sollbruch-/ -rissabschnitt, an welchem die Verschlusskappe bei deren Entfernen von dem kartuschenförmigen Behälter/ der Kartusche vordefiniert reißen oder brechen soll, an dem Flanschabschnitt vorgesehen ist. Dadurch kann ein Anwender einfacher und schneller als bei einem an einer Mantelfläche/ einem Mantelflächenabschnitt vorgesehenen Sollbruch-/ -rissabschnitt erkennen, ob der kartuschenförmige Behälter/ die Kartusche noch original verschlossen ist oder nicht, was mit einer verbesserten Handhabung des Behälters/ der Kartusche einhergeht. Insbesondere bei axialer Betrachtung des kartuschenförmigen Behälters/ der Kartusche wird somit ein leichteres Erkennen eines vorliegenden Originalitätsverschlusses ermöglicht.

In vorteilhafter Weise ist der Sollbruch-/ -rissabschnitt des Flanschabschnitts an einem Übergangsbereich zu dem Mantelflächenabschnitt vorgesehenen und als zumindest eine Sollbruchstelle und/oder eine Sollrisslinie ausgebildet, an welcher die Verschlusskappe beim Entfernen der Verschlusskappe von dem kartuschenförmigen Behälter/ der Kartusche derart abreißt und/oder bricht, dass der Versiegelungsabschnitt der Verschlusskappe an dem kartuschenförmigen Behälter/ der Kartusche verbleibt und die übrige Verschlusskappe bestehend aus Mantelflächen-/ und Deckflächenabschnitt von dem kartuschenförmigen Behälter/ der Kartusche lösbar ist.

Der Sollbruch-/ -rissabschnitt kann somit als durchgehende, umlaufende, insbesondere perforierte oder durchlochte, Sollrisslinie oder als eine Sollbruchstelle/ eine Vielzahl von Sollbruchstellen ausgebildet sein. Insbesondere stellt der Sollbruch-/ -rissabschnitt einen geschwächten Abschnitt des Flanschabschnitts dar. Dies wird generell dadurch erreicht, dass an dem Sollbruch-/ -rissabschnitt weniger Material/ eine geringere Wandstärke/ Perforationen/ Ausnehmungen/ Löcher oder Ähnliches vorgesehen sind. Dies hat den Effekt, dass bei einem Aufbringen einer Kraft zum Entfernen der Verschlusskappe ein definierter Riss/ Bruch an einer vorbestimmten Stelle an dem Flanschabschnitt erfolgt und gleichzeitig der Versiegelungsabschnitt weiterhin fixiert/ befestigt an dem kartuschenförmigen Behälter/ der Kartusche verbleibt.

Erfindungsgemäß ist somit eine erforderliche Kraft zum Herausreißen/ Entfernen des Versiegelungsabschnitts von dem kartuschenförmigen Behälter/ der Kartusche größer als eine Kraft, welche zum Reißen/ Brechen der Verschlusskappe an dem Sollbruch-/ - rissabschnitt vonnöten ist. Ein Anwender weiß somit, dass die Verschlusskappe nur an einer vorbestimmten Stelle brechen bzw. reißen kann bzw. sollte, wodurch eine Sichtprüfung in großem Maße erleichtert wird. Vor diesem Hintergrund kann ein Anwender auch darauf schließen, dass der kartuschenförmige Behälter/ die Kartusche vor dem Öffnen desselben/ derselben in geeigneter Weise versiegelt war, wenn der Siegelring/ Versiegelungsabschnitt nach dem Öffnen des Behälters noch fest befestigt/ fixiert an dem kartuschenförmigen Behälter/ der Kartusche/ dem Anschlussport vorliegt.

Ein vorteilhaftes Ausführungsbeispiel ist dadurch gekennzeichnet, dass der Versiegelungsabschnitt eine Vielzahl von Ausnehmungen aufweist, und zwar derart, dass er aus einem umlaufenden Versiegelungsringabschnitt und einer Vielzahl von, insbesondere dreieckförmigen, Verbindungsabschnitten besteht, wobei die Verbindungsabschnitte punktuell mit dem Mantelflächenabschnitt verbunden sind und der Sollbruch-/ -rissabschnitt durch die Vielzahl an punktuellen Verbindungen ausgebildet ist.

Gemäß einer bevorzugten erfindungsgemäßen Ausführungsform ist somit ein umlaufender Ringabschnitt/ Versiegelungsringabschnitt an dem kartuschenförmigen Behälter/ der Kartusche/ dem Anschlussport fixiert/ befestigt. Dieser Ringabschnitt befindet sich an einer radialen Außenseite des Flanschabschnitts. Von dem Ringabschnitt erstrecken sich viele, insbesondere acht, dreieckförmige Verbindungsabschnitte zu dem Mantelflächenabschnitt hin und kontaktieren den Mantelflächenabschnitt punktuell mit einer Dreiecksspitze. Dadurch entstehen viele, insbesondere acht, punktuelle Verbindungen zwischen dem Flanschabschnitt und dem Mantelflächenabschnitt. Die Gesamtheit der punktuellen Verbindungen dieser Ausführungsform bildet den erfindungsgemäßen Sollbruch-/ -rissabschnitt. Wird die Verschlusskappe dieser Ausführungsform gedreht und/ oder an dieser gezogen, reißen/ brechen die punktuellen Verbindungen, so dass die Verschlusskappe, nunmehr bestehend aus Mantelflächenabschnitt und Deckflächenabschnitt, abgezogen werden kann und der Versiegelungsabschnitt an dem kartuschenförmigen Behälter/ der Kartusche/ dem Anschlussport verbleibt.

Außerdem kann es vorgesehen sein, dass der Sollbruch-/ -rissabschnitt als eine perforierte, insbesondere durchlochte, umlaufende Sollrisslinie, insbesondere Perforationssollrisslinie, ausgebildet ist.

Gemäß einer zweiten bevorzugten erfindungsgemäßen Ausführungsform ist der Flanschabschnitt somit von dem Mantelflächenabschnitt bis hin zu seiner radialen Außenseite als durchgehender Vollmaterialabschnitt ausgebildet und weist lediglich an oder nahe einem Übergangsbereich zu dem Mantelflächenabschnitt eine perforierte Sollrisslinie auf. In anderen Worten wird der Sollbruch-/ -rissabschnitt gemäß dieser Ausführungsform durch viele, eng aneinander gereihte/ liegende Perforationen/ kleine Löcher ausgebildet.

Ferner kann erfindungsgemäß der Sollbruch-/ -rissabschnitt auch durchgehend undurchlocht bzw. unperforiert, beispielsweise durch eine verringerte Materialdicke, ausgebildet sein. Eine derartige Ausführungsform hat eine verbesserte Abdichtung bzw. Abdichtwirkung zur Folge. Der Sollbruch-/ rissabschnitt stellt jedoch in jedem Fall - wie in allen Ausführungsformen der vorliegenden Erfindung - eine zumindest abschnittsweise Schwachstelle an dem Flanschabschnitt der Verschlusskappe dar.

Es kann außerdem vorgesehen sein, dass sowohl der Flanschabschnitt als auch der Mantelflächenabschnitt jeweils zumindest einen Sollbruch-/ -rissabschnitt aufweisen und die Sollbruch-/ -rissabschnitte des Flanschabschnitts und des Mantelflächenabschnitts umlaufend ineinander übergehen. Gemäß dieser Ausführungsform ist somit eine umlaufende Sollrisslinie vorgesehen, welche abwechselnd von dem Flanschabschnitt zu dem Mantelflächenabschnitt übergeht und umgekehrt. Weiter bevorzugt liegt genau ein Sollbruch-/ -rissabschnitt an dem Flanschabschnitt vor, welcher nach einer 180°-Drehung zu einem Sollbruch-/ - rissabschnitt an dem Mantelflächenabschnitt übergeht. Nach einer 180°-Drehung des Sollbruch-/ -rissabschnitts an dem Mantelflächenabschnitt erfolgt wieder ein Übergang zu dem Sollbruch-/ -rissabschnitt an dem Flanschabschnitt.

Es ist von Vorteil, wenn die Verschlusskappe als eine Aufsteckkappe ausgeführt ist und der Versiegelungsabschnitt des Flanschabschnitts formschlüssig in einer an dem kartuschenförmigen Behälter/ der Kartusche vorgesehenen Aufnahme, insbesondere durch Bördeln oder Einpressen, aufgenommen und/oder an dem kartuschenförmigen Behälter/ der Kartusche befestigt ist.

Dadurch dass die Verschlusskappe als eine Aufsteckkappe ausgeführt ist, ist kein aufwändiges Aufschrauben derselben vonnöten. Eine Handhabung wird dadurch vereinfacht. Wird der Versiegelungsabschnitt, welcher insbesondere als ein Brechsiegel ausgebildet ist, formschlüssig in einer an dem kartuschenförmigen Behälter/ der Kartusche/ dem Anschlussport vorgesehenen Aufnahme aufgenommen/ befestigt, kann ein Fixieren/ Versiegeln der Verschlusskappe an dem kartuschenförmigen Behälter/ der Kartusche in einem einzigen Prozessschritt, vorzugsweise vollautomatisch, erfolgen.

Bevorzugt wird die Verschlusskappe im Rahmen einer vollautomatischen Produktionsanlage an dem kartuschenförmigen Behälter/ der Kartusche befestigt. Dabei wird die Verschlusskappe in einem ersten Schritt auf den Anschlussport aufgesteckt. In einem zweiten Schritt wird der Versiegelungsabschnitt mit Hilfe eines Einpresswerkzeugs/ eines umformenden/ umwölbenden Werkzeugs, insbesondere Bördelwerkzeugs, in die Aufnahme gedrückt/ gepresst/ umgeformt/ eingerastet/ geschweißt/ aufgeklipst und damit formschlüssig in der Aufnahme aufgenommen. Außerdem kann der Formschluss durch ein Umformen von Abschnitten des kartuschenförmigen Behälters/ der Kartusche/ des Anschlussports und ein dadurch bewirktes Einklemmen des Versiegelungsabschnitts bewirkt werden.

Es sei angeführt, dass erfindungsgemäß eine formschlüssige Verbindung des Versiegelungsabschnitt mit der an dem kartuschenförmigen Behälter/ der Kartusche vorgesehenen Aufnahme durch Bördeln oder Einpressen zwar bevorzugt ist, jedoch auch eine stoffschlüssige Verbindung durch Schweißen oder eine kraft- und formschlüssige Verbindung durch Nieten vorgesehen sein kann. Insbesondere soll eine von vielen denkbaren Verbindungsarten gewählt werden, welche die Verschlusskappe geeignet an dem kartuschenförmigen Behälter/ der Kartusche/ dem Anschlussport befestigen und versiegeln kann und die Verschlusskappe gegen ein Abrutschen bzw. Lösen von dem Anschlussport schützt.

Außerdem ist es zweckmäßig, wenn die Verschlusskappe an einer Verschlusskappeninnenseite an dem Deckflächenabschnitt und/oder dem Mantelflächenabschnitt zumindest ein Dichtelement zum Abdichten des Anschlussports des kartuschenförmigen Behälters aufweist.

Um das geschlossene Kartuschensystem der vorliegenden Erfindung bereitstellen zu können, ist es essentiell, für eine geeignete Abdichtung nach außen zu sorgen, damit die Haltbarkeit des kartuschenförmigen Behälters/ der Kartusche unabhängig von der Klimazone ist, in welcher sie verwendet wird. Bei einer erhöhten Außentemperatur, Druck und Luftfeuchtigkeit kann nämlich bei Bikarbonatkartuschen der Karbonatwert schneller ansteigen, was mit einer verkürzten Haltbarkeit einhergehen würde. Daher ist die Abdichtung ein grundlegender Aspekt der vorliegenden Erfindung.

Die Abdichtung wird erfindungsgemäß durch einstückig/ einmaterialig an einer Verschlusskappeninnenseite angeformte Dichtelemente realisiert. **In** anderen Worten werden die Dichtelemente durch eine geeignete konstruktive Gestaltung bzw. Materialauswahl der erfindungsgemäßen Verschlusskappe ausgebildet. Bevorzugt ist zumindest eine umlaufende Dichtlippe an dem Mantelflächenabschnitt an der Verschlusskappeninnenseite vorgesehen, welche zum Beispiel durch ein abschnittsweises Erhöhen/ Vergrößern der Wandstärke des Mantelflächenabschnitts konstruktiv umsetzbar ist. Es können jedoch auch zwei, drei, vier oder mehr umflaufende Dichtlippen bzw. Dichtelemente an dem Mantelflächenabschnitt vorgesehen sein.

Erfindungsgemäß sind verschiedene Ausführungsarten von Dichtelementen denkbar. Wichtig ist jedoch, dass die Dichtelemente einstückig/ einmaterialig, insbesondere im Spritzgussverfahren, in einem Prozessschritt mit der Verschlusskappe gefertigt werden und an einer Verschlusskappeninnenseite angeformt sind. Insbesondere soll eine Funktionselastizität und Materialelastizität der Dichtelemente durch deren Geometrie und dem verwendeten Material erzeugt bzw. bereitgestellt werden.

Eine Dichtwirkung kann erfindungsgemäß auch durch ein an dem Deckflächenabschnitt angeformtes, stopfenartiges, zentrales Mittelteil, welches einstückig und einmaterialig mit der Verschlusskappe ausgeführt ist und ebenso an der Verschlusskappeninnenseite vorgesehen ist, realisiert werden. Die Verschlusskappe soll erfindungsgemäß den Anschlussport lediglich durch eine Steck-/ Aufsteckverbindung und somit ohne Gewinde abdichten.

In vorteilhafter Weise sind an dem Deckflächenabschnitt an einer Verschlusskappeninnenseite zumindest ein, vorzugsweise zumindest zwei, stegartige Vorsprünge vorgesehen, welche in einem montierten Zustand der Verschlusskappe einen Auflageflächenabschnitt der Verschlusskappe auf einer Stirnfläche des, insbesondere rohrstutzenförmigen, Anschlussports darstellen.

Durch die an dem Deckflächenabschnitt der Verschlusskappe vorgesehenen und somit oben liegenden, radialen Kunststoffstege, welche vorzugsweise schmal/ langgestreckt ausgebildet und einstückig/ einmaterialig an der Verschlusskappe angeformt sind, wird eine geeignete Stoßdämpfung bereitgestellt und somit der Anschlussport des kartuschenförmigen Behälters/ der Kartusche in deutlich verbesserter Weise geschützt. Es hat sich in Versuchsreihen herausgestellt, dass dadurch, dass lediglich einzelne stegartige Vorsprünge der Verschlusskappe an der Stirnfläche des Anschlussports anliegen, die Schutzwirkung und Stoßdämpfung der Verschlusskappe deutlich verbessert wird und somit eine Beschädigung des Anschlussports verhindert wird.

Ferner ist es von Vorteil, wenn der Mantelflächenabschnitt der Verschlusskappe an einer Verschlusskappenaußenseite zumindest einen flügelförmig ausgebildeten, vorstehenden Griffabschnitt zur verbesserten Handhabung der Verschlusskappe aufweist.

Flügelförmig ausgebildete, vorstehende bzw. hervorragende Griffabschnitte verbessern eine Handhabbarkeit und damit ein Abziehen der Kappe/ Verschlusskappe. Es können dabei einer oder mehrere flügelförmige Griffabschnitte vorgesehen sein. Neben flügelförmigen Vorsprüngen sind auch kreuzförmige Vorsprünge erfindungsgemäß denkbar. Außerdem kann eine Deckflächen- bzw. Mantelflächenaußenseite der Verschlusskappe geriffelt oder gummiert ausgebildet sein, um eine Griffigkeit der Kappe zu verbessern.

Es ist zweckmäßig, wenn die Verschlusskappe aus einem elastischen Kunststoffmaterial, insbesondere einem Elastomer oder einem thermoplastischen Elastomer, ausgebildet ist und/oder als einstückiges und einmaterialiges im Spritzgussverfahren hergestelltes Kunststoffbauteil ausgebildet ist.

Elastische Materialeigenschaften der Verschlusskappe sind dabei erfindungsgemäß in vielerlei Hinsicht vonnöten und stellen somit ebenfalls einen zentralen Aspekt der vorliegenden Erfindung dar. Insbesondere wird dadurch sowohl eine Dichtigkeit der Verschlusskappe verbessert, als auch ein optimierter Schutz des Anschlussports erreicht, als auch eine stoßdämpfende Wirkung der Verschlusskappe verbessert.

Die gesamte Verschlusskappe wird vorzugsweise in einem einzigen Prozessschritt als einmaterialiges und einstückiges bzw. integrales Kunststoffbauteil im Spritzgussverfahren hergestellt.

Gemäß einem weiteren Aspekt der vorliegenden Erfindung kann zusätzlich ein Etikett abschnittsweise an der Verschlusskappe und abschnittsweise an dem kartuschenförmigen Behälter/ der Kartusche/ dem Anschlussport vorgesehen/ angebracht sein, über welches eine zusätzliche Versiegelung erfolgt und ein vorliegender Originalitätsverschluss auf den ersten Blick sichtbar wird.

Außerdem betrifft die Erfindung einen kartuschenförmigen Behälter/ eine Kartusche für eine extrakorporale Blutbehandlungsmaschine, welcher/ welche zumindest einen einen Fluideinlass und/oder einen Fluidauslass bildenden, insbesondere rohrstutzenförmigen, Anschlussport/ Konnektor aufweist, und zumindest einer Verschlusskappe wie voranstehend beschrieben zum Verschließen und Versiegeln des zumindest einen Anschlussports.

Die Erfindung wird nachfolgend mit Hilfe von Figuren weiter erläutert. Es zeigen:
- Fig. 1: eine perspektivische Ansicht einer Verschlusskappenaußenseite der erfindungsgemäßen Verschlusskappe;
- Fig. 2: eine perspektivische Ansicht einer Verschlusskappeninnenseite der erfindungsgemäßen Verschlusskappe;
- Fig. 3: eine perspektivische Ansicht einer an einem Anschlussport eines kartuschenförmigen Behälters/ einer Kartusche fixierten erfindungsgemäßen Verschlusskappe;
- Fig. 4: eine perspektivische Schnittansicht der an dem Anschlussport des kartuschenförmigen Behälters/ der Kartusche fixierten erfindungsgemäßen Verschlusskappe;
- Fig. 5: eine perspektivische Ansicht einer zweiten Ausführungsform der erfindungsgemäßen Verschlusskappe; und
- Fig. 6: eine perspektivische Schnittansicht der Verschlusskappe der Fig. 5.

Die Figuren sind lediglich schematischer Natur und dienen ausschließlich dem Verständnis der Erfindung. Gleiche Elemente sind dabei mit denselben Bezugszeichen versehen. Die Merkmale der einzelnen Ausführungsformen/ -beispiele können untereinander ausgetauscht werden.

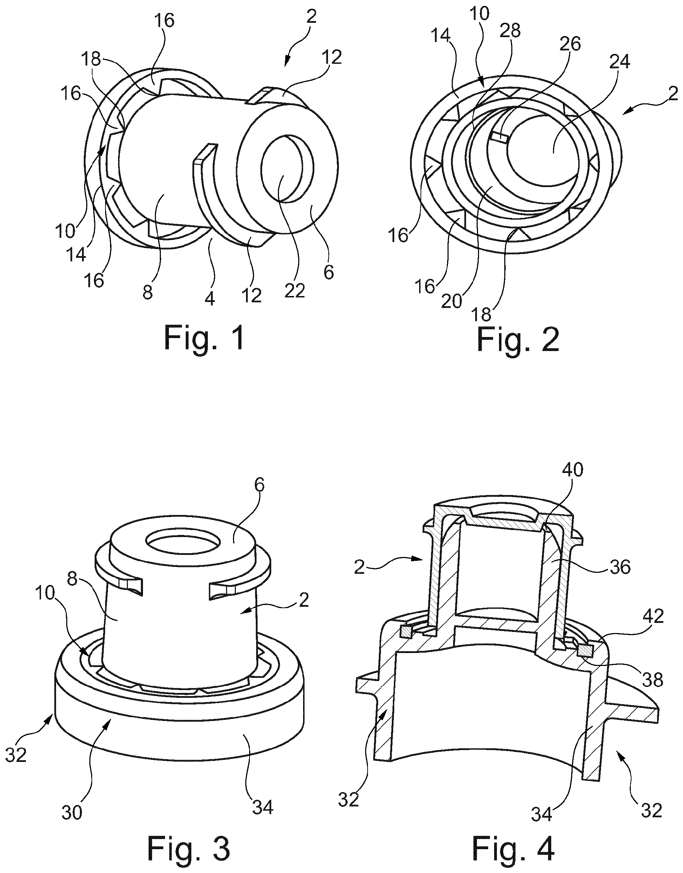

In Fig. 1 ist eine perspektivische Ansicht einer Verschlusskappe 2, insbesondere einer Verschlusskappenaußenseite 4, gezeigt. Die Verschlusskappe 2 setzt sich zusammen aus einem runden Deckflächenabschnitt 6, einem sich an den Deckflächenabschnitt 6 senkrecht und zylindrisch anschließenden Mantelflächenabschnitt 8 und einem sich an dem Mantelflächenabschnitt 8 radial senkrecht nach außen anschließenden, in Umfangsrichtung vollständig umlaufenden Flanschabschnitt 10. An einem oberen Abschnitt des Mantelflächenabschnitts 8 sind zwei flügelförmige Griffabschnitte 12 vorgesehen, welche voneinander beabstandet, einander gegenüberliegend und jeweils etwa einen Drittel- bzw. Viertelkreis einnehmend angeordnet sind. Die beiden flügelförmigen Griffabschnitte 12 stehen bezüglich des Mantelflächenabschnitts 8 vor/ ragen hervor und bilden jeweils einen teilkreisförmigen Vorsprung aus.

Der Flanschabschnitt 10 besteht in dieser bevorzugten erfindungsgemäßen Ausführungsform aus einem Versiegelungsabschnitt/ Siegelring 14 und acht dreieckigen Verbindungsabschnitten 16, welche jeweils mit einer Dreiecksseite an dem Siegelring 14 einstückig anliegend angeformt, mit einer gegenüberliegenden Dreiecksspitze auf den Mantelflächenabschnitt 8 zulaufend ausgebildet sind und um den Umfang des Flanschabschnitts gleichmäßig verteilt sind. Die acht Dreiecksspitzen gehen punktuelle Verbindungen/ Punktverbindungen 18 mit dem Mantelflächenabschnitt 8 ein. Durch die Gesamtheit der acht punktuellen Verbindungen 18 wird in der vorliegenden Ausführungsform der erfindungsgemäße Sollbruch-/ - rissabschnitt realisiert. Der Sollbruch-/ -rissabschnitt befindet sich somit an dem Flanschabschnitt 10. Der Deckflächenabschnitt 6 weist an der Verschlusskappenaußenseite 4 ein zentrales Sackloch 22 auf.

In Fig. 2 ist eine perspektivische Ansicht der Verschlusskappe 2, insbesondere einer Verschlusskappeninnenseite 20 gezeigt. Es wird hier zunächst deutlich, dass sich das in Fig. 1 gezeigte Sackloch 22 an der Verschlusskappeninnenseite 20 als stopfenartiger Vorsprung 24 ausbildet. Der Deckflächenabschnitt 6 weist an der Verschlusskappeninnenseite 20 zwei Stegabschnitte/ stegartige Vorsprünge 26 auf, von denen in Fig. 2 jedoch nur einer sichtbar ist. Die Stegabschnitte 26 sind schmal und langgestreckt ausgebildet und erstrecken sich von einer radialen Außenseite des Deckflächenabschnitts 6 hin zu dem stopfenartigen Vorsprung 24. Die Stegabschnitte 26 sind bezüglich des Deckflächenabschnitts 6 hervorstehend/ vorragend ausgebildet. Der zweite, nicht sichtbare Stegabschnitt 26 befindet sich gegenüberliegend zu dem ersten Stegabschnitt 26. Die beiden Stegabschnitte 26 sind somit um den Umfang des Deckflächenabschnitts 6 gleichmäßig verteilt angeordnet.

An einem Mittelabschnitt des Mantelflächenabschnitts 8 ist an der Verschlusskappeninnenseite 20 ein umlaufender Dichtabschnitt 28 vorgesehen, welcher nach Art einer Dichtlippe ausgebildet ist. Es können jedoch auch zwei, drei, vier oder mehr umlaufende Dichtabschnitte 28 zur Verbesserung der Dichtwirkung vorgesehen sein.

Die Verschlusskappe 2 ist in einem Prozessschritt einstückig und einmaterialig im Spritzgussverfahren aus einem elastischen Kunststoff hergestellt.

Fig. 3 zeigt eine perspektivische Ansicht einer an einem Anschlussport 30 eines kartuschenförmigen Behälters/ einer Kartusche fixierten Verschlusskappe 2. Der Anschlussport 30 weist hier einen sockelartigen Abschnitt 34 und einen rohrstutzenförmigen Abschnitt 36 auf. Die Verschlusskappe 2 ist auf den rohrstutzenförmigen Abschnitt 36 des Anschlussports 30 aufgesteckt und über ihren Siegelring 14 in einer Aufnahme 38 des sockelartigen Abschnitts 34 aufgenommen bzw. an dem sockelartigen Abschnitt 34 des Anschlussports 30 befestigt.

In dem in Fig. 3 gezeigten Zustand, in welchem die Verschlusskappe 2 in einem original verschlossenen Zustand an dem Anschlussport 30 befestigt ist, sieht ein Anwender sofort, insbesondere bei einer axialen Betrachtung der Verschlusskappe 2 in Fig. 3, dass Punktverbindungen 18 zwischen den Verbindungsabschnitten 16 und dem Mantelflächenabschnitt 8 vorliegen. Es wird somit eine sofortige, unmittelbare Sichtprüfung, ob ein Originalitätsverschluss (noch) vorliegt oder nicht (mehr), erfindungsgemäß insbesondere durch diese bevorzugte Ausführungsform ermöglicht.

Fig. 4 zeigt eine perspektivische Schnittansicht der an dem Anschlussport 30 des kartuschenförmigen Behälters/ der Kartusche fixierten Verschlusskappe 2. Dabei liegen die Stegabschnitte 26 an einer Stirnfläche 40 des rohrstutzenförmigen Abschnitts 36 des Anschlussports 30 auf. Der stopfenartige Vorsprung 24 ragt in die Öffnung des Anschlussports 30 hinein und dichtet den Anschlussport 30 nach außen ab. Es ist somit gemäß dem bevorzugten Ausführungsbeispiel der vorliegenden Erfindung keine Folie zum Abdichten des Anschlussports 30 vorgesehen. Außerdem erfüllt der Dichtabschnitt 28 eine zusätzliche Dichtfunktion und liegt vollumfänglich an einer Außenseite des rohrstutzenförmigen Abschnitts 36 des Anschlussports 30 an. Erfindungsgemäß ist somit bevorzugt eine doppelte Abdichtung, durch den Dichtabschnitt 28 einerseits und durch den stopfenartigen Vorsprung 24 andererseits vorgesehen. Es ist jedoch auch denkbar, noch weitere Dichtabschnitte 28 vorzusehen, welche parallel und in axialer Richtung versetzt zu dem dargestellten Dichtabschnitt 28 angeordnet sind und ebenfalls eine vollumfängliche Abdichtung bereitstellen.

Der Siegelring 14 kann gemäß diesem bevorzugten Ausführungsbeispiel zum einen in die ringförmige Aufnahme 38 gepresst/ gerückt werden. Dabei wird ein äußerer Ringabschnitt 42 des sockelartigen Abschnitts 34 elastisch radial nach außen verdrängt und federt, nachdem der Siegelring eingerastet bzw. aufgeklipst ist, radial nach innen zurück, so dass der Siegelring dadurch formschlüssig in die ringförmige Aufnahme 38 aufgenommen ist. Zum anderen kann es gemäß diesem bevorzugten Ausführungsbeispiel auch vorgesehen sein, dass der Siegelring 14 kraftlos in die ringförmige Aufnahme eingelegt werden kann und anschließend durch Umformen bzw. Biegen des äußeren Ringabschnitts 42 radial nach innen, eine formschlüssige Fixierung realisiert wird.

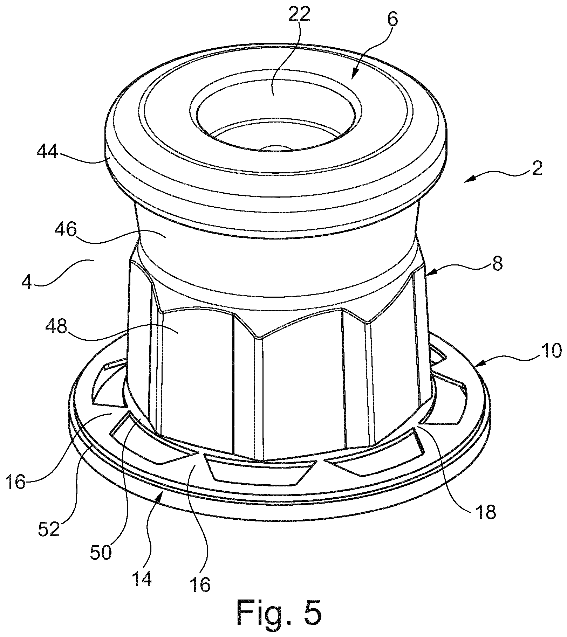

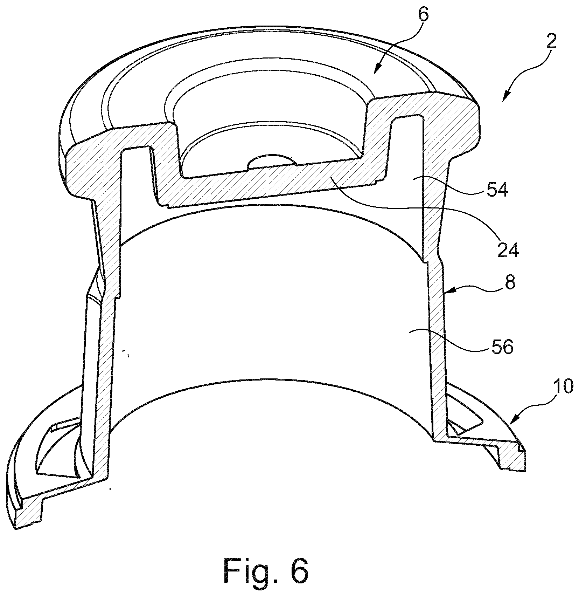

Eine zweite Ausführungsform der erfindungsgemäßen Verschlusskappe 2 ist in Fig. 5 und Fig. 6 dargestellt. Wie aus Fig. 5 hervorgeht, weist der Deckflächenabschnitt 6 in dieser Ausführungsform ebenfalls ein Sackloch 22 auf. Abgerundete Kanten zwischen dem Deckflächenabschnitt 6 und dem Sackloch 22 sowie zwischen dem Deckflächenabschnitt 6 und dem Mantelflächenabschnitt 8 verbessern eine Handhabung der Verschlusskappe 2.

Der Deckflächenabschnitt 6 geht an der Verschlusskappenaußenseite 4 in einen radial nach außen vorstehenden oberen Außenabschnitt 44 des Mantelflächenabschnitts 8 über. An den oberen Außenabschnitt 44 schließt sich nach unten ein radial zurückgenommener mittlerer Außenabschnitt 46 des Mantelflächenabschnitts 8 an. Der obere, radial vorstehende Außenabschnitt 44 kann somit in dieser Ausführungsform in einfacher Weise von einem Anwender gegriffen werden, wodurch ein Abziehen der Verschlusskappe 2 von einer Kartusche vereinfacht/ verbessert wird. Im Übrigen kann am Außenabschnitt 46 zur besseren Drehmomentaufnahme eine Schlüsselfläche (Mehrkantprofil) vorgesehen sein.

An den mittleren Außenabschnitt 44 schließt sich nach unten ein unterer geriffelter Außenabschnitt 48 des Mantelflächenabschnitts 8 an. Der untere geriffelte Außenabschnitt 48 ist eckig, beispielsweise achteckig, ausgebildet und weist konkav nach innen gewölbte Seitenabschnitte auf. Es liegt somit eine grobe Riffelung vor, durch welche ein Drehen der Verschlusskappe 2 und somit ein Öffnen der Verschlusskappe 2 vereinfacht wird.

Der Flanschabschnitt 10 der Fig. 5 weist anschließend an den geriffelten Außenabschnitt 48 einen ersten radial nach außen vorstehenden Flanschabschnitt 50 auf, welcher näherungsweise einen Umkreis um den geriffelten Außenabschnitt 48 darstellt. An den ersten radial nach außen vorstehenden Flanschabschnitt 50 schließen sich dreieckige Verbindungsabschnitte 16 an. Es ist ein erfindungsgemäßer umlaufender Sollbruch-/ -rissabschnitt zwischen dem ersten radial nach außen vorstehenden Flanschabschnitt 50 und den dreieckigen Verbindungsabschnitten 16 ausgebildet. Der sich an die dreieckigen Verbindungsabschnitte 16 anschließende Versiegelungsabschnitt 14 weist einen oberen, umlaufenden und zurückgenommenen Eckabschnitt 52 auf, in welchen beispielsweise der in Fig. 4 dargestellte Ringabschnitt 42 eingreifen kann.

In Fig. 6 wird ersichtlich, dass eine Abdichtung in dieser zweiten Ausführungsform der Verschlusskappe 2 zum einen über den stopfenartigen Vorsprung 24 und zum anderen über einen umlaufenden, oberen Innendichtabschnit 54, welcher sich von dem Deckflächenabschnitt 6 bis zu einem unteren Innenabschnitt 56 des Mantelflächenabschnitts 8 erstreckt, erfolgt.

### Bezugszeichenliste

- 2: Verschlusskappe
- 4: Verschlusskappenaußenseite
- 6: Deckflächenabschnitt
- 8: Mantelflächenabschnitt
- 10: Flanschabschnitt
- 12: flügelförmige Griffabschnitte
- 14: Siegelring
- 16: Verbindungsabschnitt
- 18: Punktverbindung
- 20: Verschlusskappeninnenseite
- 22: Sackloch
- 24: stopfenartiger Vorsprung
- 26: Stegabschnitt
- 28: Dichtabschnitt
- 30: Anschlussport
- 34: sockelartiger Abschnitt
- 36: rohrstutzenförmiger Abschnitt
- 38: Aufnahme
- 40: Stirnfläche
- 42: äußerer Ringabschnitt
- 44: oberer Außenabschnitt
- 46: mittlerer Außenabschnitt
- 48: unterer geriffelter Außenabschnitt
- 50: erster Flanschabschnitt
- 52: Eckabschnitt
- 54: Innendichtabschnitt
- 56: unterer Innenabschnitt

## Patentansprüche

1. Verschlusskappe (2), welche nicht zum Aufschrauben, sondern zum Aufstecken an einem Anschlussport (30) eines kartuschenförmigen Behälters vorbereitet ist und in einem montierten Zustand einem Verschließen des Anschlussports (30) dient, mit
einem runden Deckflächenabschnitt (6);
einem sich senkrecht an den Deckflächenabschnitt (6) anschließenden, im Wesentlichen zylindrisch verlaufenden Mantelflächenabschnitt (8); und
einem sich an den Mantelflächenabschnitt (8) anschließenden, sich endseitig des Mantelflächenabschnitts von dem Mantelflächenabschnitt senkrecht und radial nach außen weg erstreckenden Flanschabschnitt (10),
**dadurch gekennzeichnet, dass**
der Flanschabschnitt (10) einen Versiegelungsabschnitt (14), über welchen die Verschlusskappe (2) an dem kartuschenförmigen Behälter fixierbar und versiegelbar ist, und einen Sollbruch-/ -rissabschnitt (18), an welchem die Verschlusskappe (2) beim Entfernen der Verschlusskappe (2) von dem kartuschenförmigen Behälter vordefiniert reißt und/oder bricht, aufweist und/oder ausbildet,
die Verschlusskappe (2) an einer Verschlusskappeninnenseite (20) an dem Mantelflächenabschnitt (8) zumindest ein einstückig angeformtes Dichtelement (28), welches als eine umlaufende Dichtlippe ausgebildet ist, zum Abdichten des Anschlussports (30) des kartuschenförmigen Behälters aufweist, und
an dem Deckflächenabschnitt (6) an einer Verschlusskappeninnenseite (20) zumindest ein stegartiger Vorsprung (26) vorgesehen ist, welcher in einem montierten Zustand der Verschlusskappe (2) einen Auflageflächenabschnitt der Verschlusskappe (2) auf einer Stirnfläche (40) des Anschlussports (30) darstellt.

2. Verschlusskappe (2) nach Anspruch 1, **dadurch gekennzeichnet, dass** der Sollbruch-/ -rissabschnitt (18) des Flanschabschnitts an einem Übergangsbereich zu dem Mantelflächenabschnitt (8) vorgesehenen ist und als zumindest eine Sollbruchstelle (18) und/oder eine Sollrisslinie ausgebildet ist, an welcher die Verschlusskappe (2) beim Entfernen der Verschlusskappe (2) von dem kartuschenförmigen Behälter derart abreißt und/oder bricht, dass der Versiegelungsabschnitt (14) der Verschlusskappe (2) an dem kartuschenförmigen Behälter verbleibt und die übrige Verschlusskappe (2) bestehend aus Mantelflächenabschnitt (8) und Deckflächenabschnitt (6) von dem kartuschenförmigen Behälter lösbar ist.

3. Verschlusskappe (2) nach Anspruch 1 oder 2, **dadurch gekennzeichnet, dass** der Versiegelungsabschnitt (14) eine Vielzahl von Ausnehmungen aufweist, derart, dass er aus einem umlaufenden Versiegelungsringabschnitt (14) und einer Vielzahl von Verbindungsabschnitten (16) besteht, wobei die Verbindungsabschnitte (16) punktuell mit dem Mantelflächenabschnitt (8) verbunden sind und der Sollbruch-/ - rissabschnitt (18) durch die Vielzahl an punktuellen Verbindungen (18) ausgebildet ist.

4. Verschlusskappe (2) nach Anspruch 1 oder 2, **dadurch gekennzeichnet, dass** der Sollbruch-/ rissabschnitt als eine perforierte, umlaufende Sollrisslinie ausgebildet ist.

5. Verschlusskappe (2) nach einem der vorhergehenden Ansprüche, **dadurch gekennzeichnet, dass** die Verschlusskappe (2) als eine Aufsteckkappe ausgeführt ist und der Versiegelungsabschnitt (14) des Flanschabschnitts (10) formschlüssig in einer an dem kartuschenförmigen Behälter vorgesehenen Aufnahme (38) aufgenommen und/oder an dem kartuschenförmigen Behälter befestigt ist.

6. Verschlusskappe (2) nach einem der vorhergehenden Ansprüche, **dadurch gekennzeichnet, dass** die Verschlusskappe (2) an einer Verschlusskappeninnenseite (20) an dem Deckflächenabschnitt (6) zumindest ein Dichtelement (24) zum Abdichten des Anschlussports (30) des kartuschenförmigen Behälters aufweist.

7. Verschlusskappe (2) nach einem der vorhergehenden Ansprüche, **dadurch gekennzeichnet, dass** der Mantelflächenabschnitt (8) der Verschlusskappe (2) an einer Verschlusskappenaußenseite (4) zumindest einen flügelförmig ausgebildeten, vorstehenden Griffabschnitt (12) zur verbesserten Handhabung der Verschlusskappe (2) aufweist.

8. Verschlusskappe (2) nach einem der vorhergehenden Ansprüche, **dadurch gekennzeichnet, dass** die Verschlusskappe (2) aus einem elastischen Kunststoffmaterial ausgebildet ist und/oder als einstückiges und einmaterialiges im Spritzgussverfahren hergestelltes Kunststoffbauteil ausgebildet ist.

9. Kartuschenförmiger Behälter für eine extrakorporale Blutbehandlungsmaschine, welcher zumindest einen einen Fluideinlass und/oder einen Fluidauslass bildenden Anschlussport (30) aufweist, und zumindest einer Verschlusskappe (2) nach einem der vorhergehenden Ansprüche zum Verschließen und Versiegeln des zumindest einen Anschlussports (30).

## Claims

1. An end cap (2) prepared not for being screwed on, but for being attached to a connecting port (30) of a cartridge-shaped receptacle and, in a mounted state, serves for sealing the connecting port (30), comprising
a round top surface portion (6);
a shell surface portion (8) adjacent to the top surface portion (6), the shell surface portion (8) extending substantially cylindrically and perpendicularly from the top surface portion (8); and
a flange portion (10) adjacent to the shell surface portion (8), the flange portion (10) extending perpendicularly and radially outwardly away from an end side of the shell surface portion (8);
**characterized in that**
the flange portion (10) includes and/or forms a sealing portion (14), by which the end cap (2) can be fixed and sealed to the cartridge-shaped receptacle, and a predetermined breaking/tearing portion (18), at which the end cap (2) tears and/or breaks in a predefined manner when the end cap (2) is removed from the cartridge-shaped receptacle,
the end cap (2) comprises, at an inside (20) of the end cap at the shell surface portion (8), at least one integrally formed sealing element (28) configured as a circumferential sealing lip for sealing the connecting port (30) of the cartridge-shaped receptacle, and
at least one web-type projection (26) is provided on the top surface portion (6) at an inside (20) of the end cap, said projection constituting, in a mounted state of the end cap (2), a bearing face portion of the end cap (2) on an end face (40) of the connecting port (30).

2. The end cap (2) according to claim 1, **characterized in that** the predetermined breaking/tearing portion (18) of the flange portion is provided at a transition area to the shell surface portion (8) and is formed as at least one predetermined breaking point (18) and/or as a predetermined tearing line at which the end cap (2) tears and/or breaks when removing the end cap (2) from the cartridge-shaped receptacle so that the sealing portion (14) of the end cap (2) remains on the cartridge-shaped receptacle and the remaining end cap (2) consisting of the shell surface portion (8) and the top surface portion (6) is detachable from the cartridge-shaped receptacle.

3. The end cap (2) according to claim 1 or 2, **characterized in that** the sealing portion (14) comprises a plurality of recesses such that the sealing portion (14) consists of a circumferential sealing ring portion (14) and a plurality of connecting portions (16), the connecting portions (16) being locally connected to the shell surface portion (8) and the predetermined breaking/tearing portion (18) being formed by the plurality of local connections (18).

4. The end cap (2) according to claim 1 or 2, **characterized in that** the predetermined breaking/tearing portion is in the form of a perforated circumferential predetermined tearing line.

5. The end cap (2) according to one of the preceding claims, **characterized in that** the end cap (2) is in the form of an attachment cap and the sealing portion (14) of the flange portion (10) is accommodated in a seating (38) provided on the cartridge-shaped receptacle and/or is fastened to the cartridge-shaped receptacle by form fit.

6. The end cap (2) according to one of the preceding claims, **characterized in that** the end cap (2) comprises at least one sealing element (24) for sealing the connecting port (30) of the cartridge-shaped receptacle at an inside (20) of the end cap on the top surface portion (6).

7. The end cap (2) according to one of the preceding claims, **characterized in that** the shell surface portion (8) of the end cap (2) comprises at least one wing-shaped projecting handle portion (12) at an outside (4) of the end cap for improved handling of the end cap (2).

8. The end cap (2) according to one of the preceding claims, **characterized in that** the end cap (2) is made from elastic plastic material and/or is in the form of a one-piece and single-material plastic component manufactured by injection molding.

9. A cartridge-shaped receptacle for an extracorporeal blood treatment machine which comprises at least one connecting port (30) forming a fluid inlet and/or a fluid outlet, and comprising at least one end cap (2) according to claim 1 for closing and sealing the at least one connecting port (30).

## Revendications

1. Capuchon de fermeture (2) qui n'est pas préparé pour le vissage mais pour l'enfichage au niveau d'un port de raccordement (30) d'un récipient en forme de cartouche et sert dans un état monté à une fermeture du port de raccordement (30) avec
une section de surface de recouvrement (6) ronde ;
une section de surface enveloppe (8) se raccordant perpendiculairement à la section de surface de recouvrement (6), s'étendant sensiblement de manière cylindrique ; et
une section de bride (10) se raccordant à la section de surface enveloppe (8), s'étendant côté extrémité de la section de surface enveloppe de la section de surface enveloppe perpendiculairement et radialement vers l'extérieur,
**caractérisé en ce que**
la section de bride (10) présente et/ou configure une section de scellage (14) par le biais de laquelle le capuchon de fermeture (2) peut être fixé et scellé au récipient en forme de cartouche, et une section de fracture/rupture de consigne (18) au niveau de laquelle le capuchon de fermeture (2) rompt et/ou se fracture lors du retrait du capuchon de fermeture (2) de manière prédéfinie du récipient en forme de cartouche,
le capuchon de fermeture (2) présente au niveau d'un côté intérieur de capuchon de fermeture (20) sur la surface de surface enveloppe (8) au moins un élément étanche (28) formé d'un seul tenant qui est réalisé comme une lèvre étanche circonférentielle pour rendre étanche le port de raccordement (30) du récipient en forme de cartouche et
au moins une saillie (26) de type nervure est prévue sur la section de surface de recouvrement (6) au niveau d'un côté intérieur de capuchon de fermeture (20), saillie qui représente dans un état monté du capuchon de fermeture (2) une section de surface d'appui du capuchon de fermeture (2) sur une surface avant (40) du port de raccordement (30).

2. Capuchon de fermeture (2) selon la revendication 1, **caractérisé en ce que** la section de rupture/fracture de consigne (18) de la section de bride est prévue au niveau d'une zone de transition vers la section de surface enveloppe (8) et est réalisée comme au moins un point de fracture de consigne (18) et/ou une ligne de rupture de consigne, au niveau de laquelle rompt et/ou se fracture le capuchon de fermeture (2) lors du retrait du capuchon de fermeture (2) du récipient en forme de cartouche, de telle manière que la section de scellage (14) du capuchon de fermeture (2) reste au niveau du récipient en forme de cartouche et le capuchon de fermeture restant (2) se composant de la section de surface enveloppe (8) et de la section de surface de recouvrement (6) peut être détaché du récipient en forme de cartouche.

3. Capuchon de fermeture (2) selon la revendication 1 ou 2, **caractérisé en ce que** la section de scellage (14) présente une pluralité d'évidements de telle manière qu'elle se compose d'une section d'anneau de scellage (14) circonférentielle et d'une pluralité de sections de liaison (16), dans lequel les sections de liaison (16) sont reliées ponctuellement à la section de surface enveloppe (8) et la section de rupture/fracture de consigne (18) est réalisée par la pluralité de liaisons (18) ponctuelles.

4. Capuchon de fermeture (2) selon la revendication 1 ou 2, **caractérisé en ce que** la section de fracture/rupture de consigne est réalisée comme une ligne de rupture de consigne circonférentielle perforée.

5. Capuchon de fermeture (2) selon l'une quelconque des revendications précédentes, **caractérisé en ce que** le capuchon de fermeture (2) est réalisé comme un capuchon d'enfichage, et la section de scellage (14) de la section de bride (10) est logée par complémentarité de formes dans un logement (38) prévu au niveau du récipient en forme de cartouche et/ou est fixée au récipient en forme de cartouche.

6. Capuchon de fermeture (2) selon l'une quelconque des revendications précédentes,**caractérisé en ce que** le capuchon de fermeture (2) présente au moins un élément étanche (24) pour rendre étanche le port de raccordement (30) du récipient en forme de cartouche au niveau d'un côté intérieur de capuchon de fermeture (20) sur la section de surface de recouvrement (6).

7. Capuchon de fermeture (2) selon l'une quelconque des revendications précédentes, **caractérisé en ce que** la section de surface enveloppe (8) du capuchon de fermeture (2) présente au niveau d'un côté extérieur de capuchon de fermeture (4) au moins une section de préhension (12) en saillie réalisée en forme d'aile pour améliorer la manipulation du capuchon de fermeture (2).

8. Capuchon de fermeture (2) selon l'une quelconque des revendications précédentes, **caractérisé en ce que** le capuchon de fermeture (2) est réalisé en un matériau plastique élastique et/ou est réalisé comme un composant en matériau plastique fabriqué dans un procédé de moulage par injection à un matériau et d'un seul tenant.

9. Récipient en forme de cartouche pour une machine de traitement extracorporel du sang qui présente au moins un port de raccordement (30) formant une entrée de fluide et/ou une sortie de fluide, et au moins un capuchon de fermeture (2) selon l'une quelconque des revendications précédentes pour la fermeture et le scellage de l'au moins un port de raccordement (30).
